# FASCICULE DE BREVET EUROPEEN

(11) **EP 3 634 175 B1**
(45) Date de publication et mention de la délivrance du brevet: **13.11.2024**
(21) Numéro de dépôt: 18728690.1
(22) Date de dépôt: 03.05.2018
(51) Int. Cl.: A45D 40/16, B29C 39/02, A45D 40/00

(54) **BATON DE PRODUIT COSMETIQUE COMPRENANT DEUX COMPOSITIONS AYANT UNE SURFACE EN RELIEF, PROCEDE ET DISPOSITIF DE FABRICATION D'UN TEL BATON DE PRODUIT COSMETIQUE**
STIFT EINES KOSMETIKPRODUKTS MIT ZWEI ZUSAMMENSETZUNGEN MIT EINER RELIEFARTIGEN OBERFLÄCHE, VERFAHREN UND VORRICHTUNG ZUR HERSTELLUNG SOLCH EINES STIFTS EINES KOSMETIKPRODUKTS
STICK OF COSMETIC PRODUCT COMPRISING TWO COMPOSITIONS HAVING A SURFACE IN RELIEF, METHOD AND DEVICE FOR MANUFACTURING SUCH A STICK OF COSMETIC PRODUCT

(30) Priorité: 09.05.2017 FR 1754064
(43) Date de publication de la demande: 15.04.2020
(73) Titulaire: Parfums Christian Dior, 75008 Paris (FR)
(72) Inventeur: CHARTIER, Thierry, 45380 Chaingy (FR); BARRAULT, Rémi, 45800 Saint Jean De Braye (FR)
(74) Mandataire: Plasseraud IP
(86) Numéro de dépôt international: PCT/FR2018/051117
(87) Numéro de publication internationale: WO 2018/206883

(56) Documents cités:
- EP-A1- 0 712 593
- WO-A2-2012/161937
- FR-A1- 2 911 258
- FR-A3- 2 823 082
- JP-A- 2000 300 338
- US-A- 4 740 097

## Description

La présente invention est relative à des bâtons de produit cosmétique notamment de rouge à lèvre ou de produit pour le teint, comprenant deux compositions et présentant une portion de surface décorative en relief.

Plus particulièrement, l'invention se rapporte à un bâton de produit cosmétique comprenant deux compositions, selon la revendication 1, à un procédé de fabrication d'un tel bâton de produit cosmétique, selon les revendications 7 et 8, et à un dispositif de fabrication d'un tel bâton de produit cosmétique selon la revendication 10. A noter pour toute la suite que l'invention se rapporte à un bâton de produit cosmétique comprenant au moins deux compositions, c'est-à-dire qu'il pourrait comporter plus que deux compositions différentes.

Le document FR2417980A1 décrit un bâton de rouge à lèvre du type coeur-gaine et un dispositif de fabrication d'un tel bâton de rouge à lèvre.

Toutefois, le bâton de rouge à lèvre décrit dans FR2417980A1 ne présente pas une portion de surface décorative en relief et le dispositif décrit ne permet pas de fabriquer un bâton de rouge à lèvre du type coeur-gaine présentant une portion de surface décorative en relief. En effet, le dispositif comporte un moule rigide dans lequel le bâton de rouge à lèvre est coulé ou injecté. Un tel moule ne permet pas de réaliser une portion de surface décorative en relief sur la gaine du bâton de rouge à lèvre. En effet, lors du démoulage du bâton de rouge à lèvre, la portion de surface décorative en relief viendrait frotter contre le moule et serait donc très fortement altérée.

Le document FR2823082A3 décrit un bâton de rouge à lèvre présentant une portion de surface décorative en relief et un dispositif de fabrication d'un tel bâton de rouge à lèvre.

Toutefois, le bâton de rouge à lèvre décrit dans FR2823082A3 n'est pas du type coeur-gaine et le dispositif décrit ne permet pas de fabriquer un bâton de rouge à lèvre du type coeur-gaine présentant une portion de surface décorative en relief. En effet, le dispositif comporte un moule élastique dans lequel le bâton de rouge à lèvre est coulé ou injecté. Un tel moule ne permet pas de réaliser le coulage ou l'injection de deux compositions pour former une gaine entourant un coeur. En effet, pour ce faire, il est nécessaire d'introduire dans le moule un noyau qui vient en appui au fond du moule élastique sans que ce dernier ne se déforme. Le dispositif décrit dans FR2823082A3 ne permet pas de garantir cet appui.

Selon l'abrégé de WO2012161937A2, un rouge à lèvres, un brillant à lèvres et un crayon à lèvres sont disposés en un seul corps en forme de balle. Le brillant à lèvres forme un noyau du corps et le rouge à lèvres forme une gaine qui enveloppe coaxialement le noyau du brillant à lèvres. Le corps a une partie de revêtement de lèvre supérieure qui s'étend le long d'un premier côté, et une partie de revêtement de lèvre inférieure qui s'étend le long du deuxième côté du corps opposé au premier côté. La section transversale du corps est en forme de pendentif. Une moitié supérieure de la forme pendentif est en forme de coin et définit un sommet. Une moitié inférieure de la forme pendentif est de forme semi-circulaire et définit une partie arrondie. La partie de revêtement de lèvre supérieure est positionnée dans l'apex et la partie de revêtement de lèvre inférieure est positionnée au centre de la partie arrondie.

Selon l'abrégé de JP2000300338A, un dispositif de moulage d'un bâton de cosmétique est composé d'une matrice de remplissage composée d'une partie de remplissage cylindrique avec une extrémité supérieure fermée faisant saillie vers l'extrémité supérieure d'un cylindre de maintien destiné à tenir un bâton de cosmétique formé de un matériau mou.

Selon l'abrégé de FR2911258A1, un dispositif de fabrication de bâton de rouge à lèvres inclut un moule élastique ayant une cavité de moulage pour remplir et mouler un élément de bâton de rouge à lèvres, au moins trois moules incurvés disposés de façon mobile au-dessus du moule élastique pour former un trou quand les moules incurvés sont déplacés les uns vers les autres de façon radiale vers l'intérieur ou l'extérieur, et pour recevoir le matériau du rouge à lèvre pour former une portion de base de l'élément du bâton de rouge à lèvres. La portion de base de l'élément du bâton de rouge à lèvres peut être exposée quand les moules incurvés sont déplacés en s'écartant les uns des autres afin de permettre au curseur du corps tubulaire du bâton de rouge à lèvres d'être facilement et rapidement engagé sur la portion de base de l'élément du bâton de rouge à lèvres.

Selon l'abrégé de US4740097A, un bâton de rouge à lèvres comprend une mine de crayon à lèvres en contact intime avec une masse de rouge à lèvres. La mine de crayon à lèvres s'étend le long d'une partie de la périphérie extérieure du rouge à lèvres, en butée contre la masse de rouge à lèvres le long d'une ligne de démarcation s'étendant entre deux points espacés sur la périphérie du bâton de rouge à lèvres. La mine de crayon à lèvres est formée d'une seule pièce avec le rouge à lèvres de sorte que lors d'un mouvement transversal du bâton de rouge à lèvres sur la lèvre, deux zones contiguës sont produites. Une zone ne comprend que la mine de crayon à lèvres et l'autre zone ne comprend que le rouge à lèvres.

La présente invention a notamment pour but de réaliser un bâton de produit cosmétique comprenant deux compositions et qui présente une portion de surface décorative en relief.

A cet effet, le bâton de produit cosmétique selon l'invention, notamment de rouge à lèvre, comprend deux compositions A et B s'étendant le long d'un axe longitudinal, le bâton de produit cosmétique ayant une surface latérale présentant une portion de surface décorative en relief.

Dans des modes de réalisation préférés de l'invention, on peut éventuellement avoir recours en outre à l'une et/ou à l'autre des dispositions suivantes :
Le bâton de produit cosmétique est du type coeur-gaine, les deux compositions A et B étant disposées l'une par rapport à l'autre pour former un coeur entouré d'une gaine le long de l'axe longitudinal, la gaine ayant une surface latérale présentant la portion de surface décorative en relief ;
Le bâton de produit cosmétique a une extrémité libre biseautée ;
La gaine présente au niveau de l'extrémité libre biseautée une épaisseur constante autour du coeur ;
Au niveau de l'extrémité libre biseautée le coeur est concentrique avec la gaine ;
La portion de surface décorative en relief est en saillie ou en creux.

L'invention se rapporte encore, selon un autre aspect, à un procédé de fabrication d'un bâton de produit cosmétique, notamment de rouge à lèvre, comprenant deux compositions A et B, s'étendant le long d'un axe longitudinal, le bâton ayant une surface latérale présentant une portion de surface décorative en relief, caractérisé en ce que le procédé met en oeuvre :
- une étape consistant à mettre en place un noyau dans un godet présentant une portion de surface en relief,
- une étape consistant à couler ou injecter une composition A entre le noyau et le godet de sorte de former un premier élément,
- une étape consistant à retirer le noyau du godet,
- une étape consistant à couler ou injecter une composition B de sorte de former un deuxième élément, formant ainsi un bâton de produit cosmétique, et
- une étape consistant à déformer le godet de sorte de démouler le bâton de produit cosmétique.

Le procédé peut en outre comporter une étape consistant à lubrifier le noyau.

L'invention se rapporte encore, selon un autre aspect, à un dispositif de fabrication d'un bâton de produit cosmétique, notamment de rouge à lèvre, comprenant deux compositions A et B, s'étendant le long d'un axe longitudinal, le bâton ayant une surface latérale présentant une portion de surface décorative en relief.

Le dispositif selon l'invention permet en particulier la fabrication d'un bâton de produit cosmétique, notamment de rouge à lèvre, du type coeur-gaine, s'étendant le long d'un axe longitudinal, comprenant deux compositions A et B disposées l'une par rapport à l'autre pour former un coeur entouré d'une gaine le long de l'axe longitudinal, la gaine ayant une surface latérale présentant une portion de surface décorative en relief.

Dans des modes de réalisation préférés de l'invention, on peut éventuellement avoir recours en outre à l'une et/ou à l'autre des dispositions suivantes :
- Le bâton de produit cosmétique, notamment de rouge à lèvre, est du type coeur-gaine, les deux compositions A et B étant disposées l'une par rapport à l'autre pour former un coeur entouré d'une gaine le long de l'axe longitudinal, la gaine ayant une surface latérale présentant la portion de surface décorative en relief ;
- Le godet est d'une taille inférieure à celle de la cavité de façon à définir un espace entre le godet et la cavité, et le dispositif comporte un moyen pour mettre en dépression l'espace ;
- Le noyau présente un canal d'évacuation d'air débouchant d'une part sur l'extrémité du noyau en contact avec la surface interne biseautée du godet et d'autre part dans une portion du noyau hors du godet lorsque le noyau est inséré dans ledit godet ;
- Le dispositif comprend en outre un moyen d'ajustement de pression du noyau dans le godet ; le moyen d'ajustement de pression peut comporter un ressort ;
- Le dispositif comprend en outre un moyen de guidage en translation et de centrage du noyau dans le godet ; le moyen de guidage en translation et de centrage peut être escamotable ;
- Le noyau et le moyen de guidage en translation et de centrage sont montés sur un bloc ;
- Le dispositif comprend en outre un moyen d'aimantation permettant de maintenir le moyen de guidage en translation et de centrage en position escamotée ;
- Le moyen de guidage en translation et de centrage comporte une bague de centrage apte à coopérer avec un logement pourvu dans la base ;
- Le dispositif comprend une pluralité de cavités, une pluralité de godets élastiques présentant chacun une surface en relief et une pluralité de noyaux, dans lequel la pluralité de cavités est formée dans la base, chacun des godets étant reçu dans chaque cavité, et dans lequel chacun des noyaux est mobile en translation entre une position insérée dans laquelle les noyaux sont positionnés dans les godets et une position retirée dans laquelle les noyaux sont retirés des godets.

Grâce à ces dispositions, on peut réaliser un bâton de produit cosmétique, notamment de rouge à lèvre, du type coeur-gaine présentant une portion de surface décorative en relief de bonne qualité.

Le motif présent sur cette portion de surface décorative pourra notamment représenter élément décoratif et/ou informatif, tel qu'un motif décoratif, un logo, une marque de fabrique, une inscription personnalisée, etc....

D'autres caractéristiques et avantages de l'invention apparaîtront au cours de la description suivante d'une de ses formes de réalisation, donnée à titre d'exemple non limitatif, en regard des dessins joints. Sur les différentes figures, les mêmes références désignent des éléments identiques ou similaires.

Sur les dessins :
- la figure 1A est une vue de face d'un bâton de produit cosmétique selon l'invention ;
- la figure 1B est une vue de côté du bâton de produit cosmétique de la figure 1A ;
- la figure 2 est une vue éclatée en perspective du dispositif selon l'invention, illustrant le procédé selon l'invention ;
- la figure 3A est une vue en perspective du dispositif de la figure 2, les noyaux étant en-dehors des godets ;
- la figure 3B est une vue en perspective du dispositif de la figure 2, les noyaux étant insérés dans les godets ;
- la figure 4A est une vue en coupe partielle du dispositif de la figure 3B selon la ligne IV-IV, le moyen de guidage en translation et de centrage étant escamoté, les noyaux n'étant pas représentés pour une meilleure visibilité ;
- la figure 4B est une vue en coupe partielle du dispositif de la figure 3B selon la ligne IV-IV, le moyen de guidage en translation et de centrage étant déployé et au contact de la plaque de réduction, les noyaux n'étant pas représentés pour une meilleure visibilité ;
- la figure 4C est une vue en coupe partielle du dispositif de la figure 3B selon la ligne IV-IV, le moyen de guidage en translation et de centrage étant déployé et à distance de la plaque de réduction, les noyaux n'étant pas représentés pour une meilleure visibilité ;
- la figure 5 est une vue en coupe du dispositif de la figure 4 selon la ligne V-V ;
- la figure 6A est une vue en coupe de la base du dispositif de la figure 5 avec la gaine coulée dans le godet ;
- la figure 6B est une vue en coupe de la base du dispositif de la figure 5 avec le coeur coulé dans la gaine;
- la figure 7 est une vue en coupe de la base du dispositif de la figure 5 avec le coeur coulé dans la gaine ; et

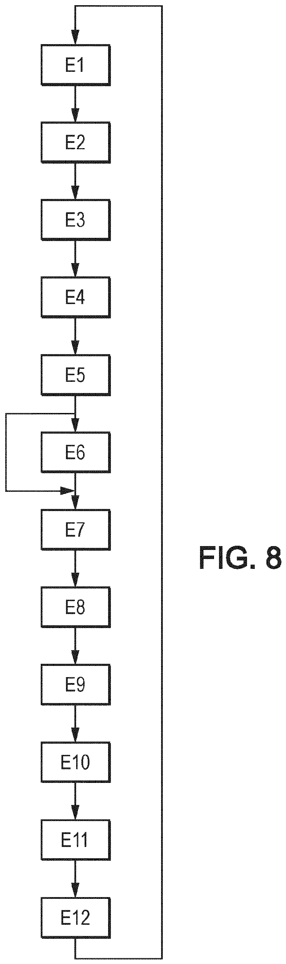

- la figure 8 est un chronogramme illustrant de façon schématique les étapes du procédé selon l'invention.

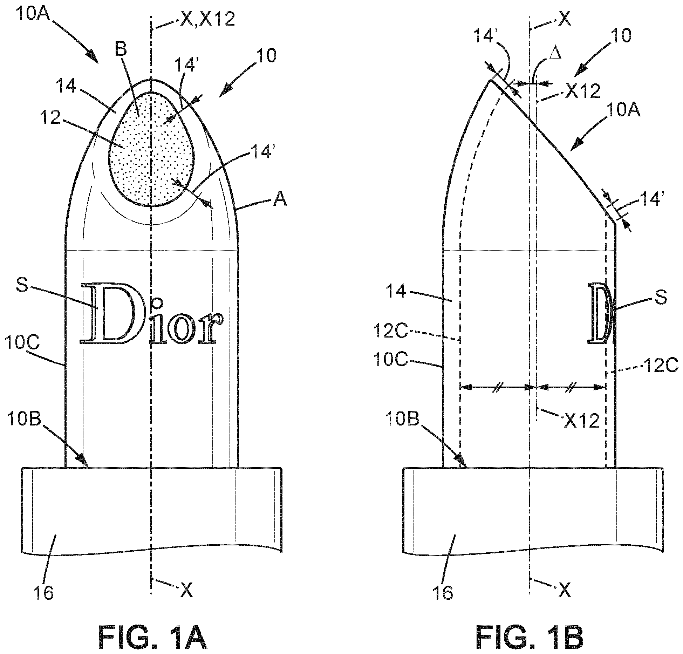

Les figures 1A et 1B représentent un bâton de produit cosmétique 10 selon l'invention. Le bâton de produit cosmétique est notamment de rouge à lèvre.

Le bâton de produit cosmétique 10 s'étend selon un axe longitudinal X-X. Le bâton de produit cosmétique 10 comprend deux compositions A et B s'étendant le long d'un axe longitudinal X-X. Le bâton de produit cosmétique 10 peut être du type coeur-gaine, comprenant deux compositions A et B disposées l'une par rapport à l'autre pour former un coeur 12 entouré d'une gaine 14 le long de l'axe longitudinal X-X. Pour toute la suite, la description est faite pour un bâton de produit cosmétique 10 du type coeur-gaine, mais il doit être entendu qu'elle n'est pas limitée à ce type de bâton de produit cosmétique, mais peut être appliquée à tout type de bâton de produit cosmétique 10 comprenant deux compositions A et B.

Les compositions A et B sont des produits cosmétiques qui entrent dans la composition des bâtons de produit cosmétique et qui sont de préférence visuellement distincts.

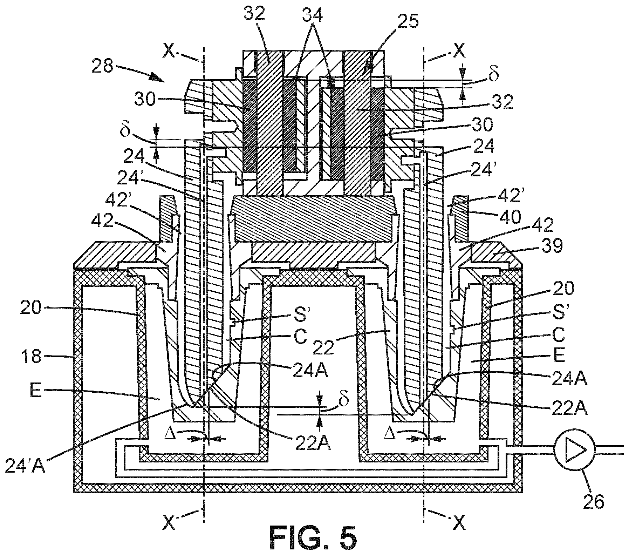

Comme mieux visible sur la figure 1A, la gaine 14 du bâton de produit cosmétique 10 présente une portion de surface décorative S en relief sur la surface latérale 10C du bâton de produit cosmétique 10. Par relief, il est entendu ici que la portion de surface décorative S est en saillie, comme illustré sur les figures 1A et 1B, ou en creux, comme illustré sur les figures 5 à 7, de manière à former au moins une contre-dépouille par rapport à un démoulage selon l'axe du bâton de produit cosmétique 10.

Le bâton de produit cosmétique 10 comporte une extrémité libre 10A et, à l'opposé de cette dernière, une extrémité 10B qui est fixée à un mécanisme distributeur 16 de type connu. L'extrémité 10A peut être en forme d'ogive. Le bâton de produit cosmétique 10 peut être biseauté au niveau de l'extrémité libre 10A, comme mieux visible sur la figure 1B. Ce biseau est décentré du fait de la forme bombé de l'extrémité en ogive du bâton de produit cosmétique 10.

Par ailleurs, la gaine 14 peut présenter au niveau de l'extrémité libre biseautée 10A une épaisseur constante autour du coeur 12 mesurée dans le plan du biseau. Plus particulièrement, au niveau de l'extrémité libre biseautée 10A, la gaine 14 présente une épaisseur 14' qui est constante tout autour du coeur 12. Par la suite sera décrit de quelle façon il est possible d'avoir cette constance dans l'épaisseur alors que l'extrémité libre 10A est biseautée.

La section transversale du bâton est de préférence circulaire, mais pourrait présenter toute autre forme.

De même la section transversale du coeur est de préférence circulaire, mais pourrait présenter toute autre forme, par exemple ovale, en goutte, polygonale, notamment en étoile.

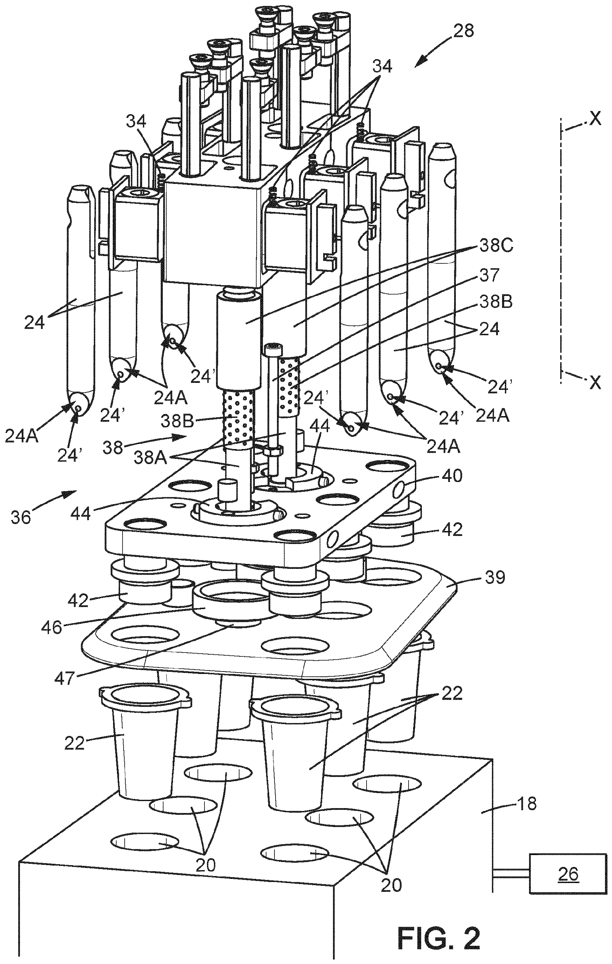

La figure 2 représente en vue éclatée le dispositif selon l'invention permettant de mettre en oeuvre le procédé selon l'invention.

Le dispositif selon l'invention permet de fabriquer un bâton de produit cosmétique 10 tel que décrit précédemment. Le dispositif permet ainsi de fabriquer un bâton de produit cosmétique 10, notamment de rouge à lèvre, comprenant deux compositions A et B, s'étendant le long d'un axe longitudinal (X-X), mais également un bâton de produit cosmétique, notamment de rouge à lèvre, du type coeur-gaine. Pour toute la suite, la description est faite d'un dispositif pour fabriquer un bâton de produit cosmétique 10 du type coeur-gaine, mais il doit être entendu qu'elle n'est pas limitée à ce type de bâton de produit cosmétique, mais peut être appliquée à tout type de bâton de produit cosmétique 10 comprenant deux compositions A et B.

Le dispositif comporte une base 18 comportant une cavité 20 formée dans la base 18. De préférence, la base 18 comporte une pluralité de cavités 20 permettant ainsi la fabrication simultanée de plusieurs bâtons de produit cosmétique 10 selon l'invention. En l'espèce, tel que représenté sur les figures, la base 18 comporte six cavités 20, mais elle pourrait en comporter un nombre différent (inférieur ou supérieur).

Le dispositif selon l'invention comporte en outre un godet 22 élastique qui est reçu dans la cavité 18. Pour des raisons de compatibilité physico-chimique avec les différentes compositions A et B, le godet 22 sera de préférence formé à partir d'un matériau présentant une élasticité nécessaire à sa déformation permettant d'extraire le bâton de produit cosmétique et ne présentant pas de risque d'interaction avec ces compositions, tel que du caoutchouc ou de la silicone.

Le godet 22 présente une surface en relief (non illustrée) qui est complémentaire à la surface en relief S réalisée sur la gaine 14. En l'espèce, comme illustré sur les figures, lorsque la surface en relief S du bâton de produit cosmétique 10 est en protubérance, la surface en relief S' du godet 22 est en renfoncement. A l'inverse, lorsque la surface en relief du bâton de produit cosmétique 10 est en creux, la surface en relief du godet 22 est en protubérance.

Tel que représenté sur les figures, le dispositif selon l'invention peut comporter six godets 22 élastiques qui sont chacun reçu dans une des six cavités 18. Chacun des godets 22 présente une surface en relief S' qui est complémentaire à la surface en relief S réalisée sur la gaine 14.

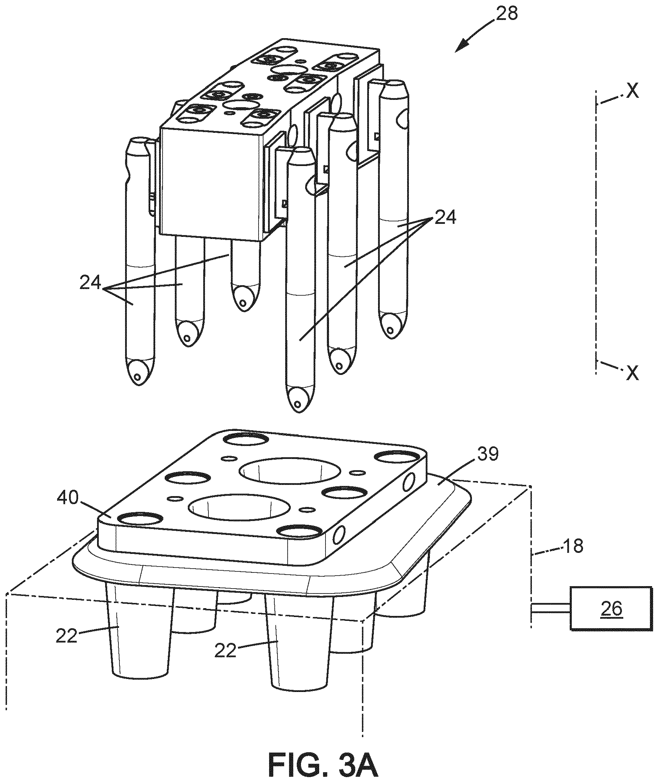

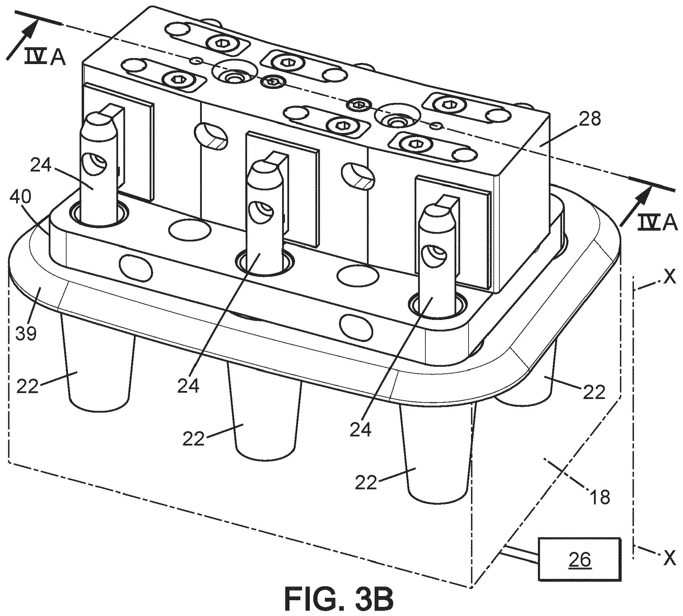

Le dispositif selon l'invention comporte en outre un noyau 24 qui est mobile en translation selon un axe longitudinal X-X entre une position insérée, tel qu'illustré à la figure 3B dans laquelle le noyau est positionné dans le godet 22 et une position retirée, tel qu'illustré à la figure 3A, dans laquelle le noyau est retiré du godet 22. Le noyau 24 est de préférence réalisé en métal, par exemple en acier ou en aluminium, pouvant présenter un revêtement réalisé par exemple par anodisation. En l'espèce, tel que représenté sur les figures, le dispositif selon l'invention comporte six noyaux 24 qui sont chacun reçu dans un des six godets 22.

Le dispositif comporte en outre un moyen 26 pour déformer le godet 22 afin de pouvoir extraire un bâton de produit cosmétique du godet sans abimer la portion de surface décorative S en relief. En l'espèce, le moyen 26 permet de déformer chacun des six godets 22.

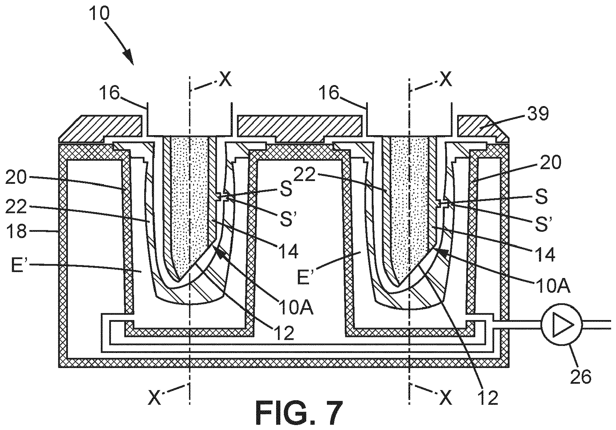

Chaque godet 22 est d'une taille inférieure à celle de la cavité 20, de façon à définir un espace E entre chacun des godets 22 et la cavité 20 correspondante. Le moyen 26 permet de mettre en dépression les espaces E et ainsi déformer chacun des godets 22 et autoriser le démoulage aisé et sans détérioration du bâton de produit cosmétique 10, tel qu'illustré sur la figure 7. Le moyen 26 est du type connu et peut comprendre une pompe à vide, des canalisations et des clapets anti-retour.

Un tel dispositif permet ainsi de réaliser simultanément six bâtons de produit cosmétique 10 selon l'invention.

Pour toute la suite, la description est faite pour un godet 22 disposé dans une cavité 20 avec un noyau 24, mais il doit être compris qu'elle s'applique également à la pluralité de godets 22 disposés dans une pluralité de cavités 20 avec une pluralité de noyaux 24.

Pour réaliser une surface biseautée 10A du coeur 12 entouré de gaine 14 au niveau de l'extrémité libre 10A, il est nécessaire que le noyau 24 vienne en contact avec le fond du godet 22. A cet effet, le noyau 24 présente une extrémité libre 24A biseauté de forme équivalente à l'extrémité libre biseautée 10A du bâton de produit cosmétique 10. Il en va de même pour l'intérieur du godet 22 qui présente également une surface interne biseautée 22A correspondante. Ainsi, lorsque le noyau 24 est inséré dans le godet 22, l'extrémité libre 24A du noyau 24 vient au contact de la surface interne biseautée 22A du godet 22. Ce contact permet d'éviter que la composition A de la gaine 14 ne passe entre l'extrémité libre 24A du noyau 24 et la surface interne biseautée 22A du godet 22; en l'absence de contact le coeur 12 ne serait pas visible au niveau de l'extrémité libre 10A car il serait intégralement recouvert par la gaine 12 avant les premières utilisations.

Par ailleurs, afin de garantir une épaisseur 14' constante de la gaine 14 autour du coeur 12 au niveau de l'extrémité libre 10A il est nécessaire de décaler le noyau 24 par rapport à l'axe longitudinal X-X, tel que mieux visible à la figure 5. A cet effet, un décalage Δ peut être prévu entre l'axe longitudinal X-X et l'axe X12-X12 du coeur 12. L'axe X12-X12 du noyau 12 est défini comme étant l'axe qui passe par le bâton de cosmétique 10 à équidistance des parois latérales 12C du coeur 12 (figure 1B). Cet axe est donc défini en dehors de la zone de la surface oblique 10A.

Le godet 22 étant élastique, afin d'éviter qu'il ne soit entrainé par aspiration lorsque le noyau 24 passe de position insérée (figure 3B) à sa position retirée (figure 3A), le noyau 24 présente un canal d'évacuation d'air 24' (ou évent) débouchant d'une part à l'extrémité libre 24A du noyau 24 sur le biseau et d'autre part dans une portion du noyau 24 hors du godet 22 lorsque le noyau 24 est inséré dans ledit godet 24 et permettant une mise à l'air du noyau 24 et évitant l'effet ventouse.

En outre, le godet 22 étant élastique, afin d'assurer le contact entre l'extrémité libre 24A du noyau 24 et la surface interne biseautée 22A du godet 22 et éviter que le godet 22 ne se déforme sous une pression trop importante du noyau 24, le dispositif comporte un moyen d'ajustement de pression 25 du noyau 24 dans le godet 22.

A cet effet, le noyau 24 est monté coulissant sur un bloc 28 par l'intermédiaire d'un moyen d'ajustement de pression 25 comportant un palier 30 et une tige 32 ; le moyen d'ajustement de pression 25 du noyau 24 comporte en outre un ressort 34 permettant d'exercer une pression sur le palier 30 et ainsi réguler la pression exercée par noyau 24 dans le fond du godet 22. Les caractéristiques du ressort 34 sont choisies en fonction des dimensions du noyau 24 et du godet 22 et de l'élasticité du godet 22. L'élasticité du ressort 34 est adaptée pour que le déplacement du doigt par rapport au noyau 24 soit stoppé avant la déformation du noyau 24 alors que le déplacement du bloc 28 poursuit sa course de mise en place. Le ressort 34 est de préférence un ressort de compression.

La course de coulissement du noyau est inférieure à 5 mm, par exemple égale à 2 mm. Lorsque le noyau 24 est correctement en place dans le godet 22 le moyen d'ajustement de pression 25 est avantageusement sensiblement à mi-course.

Chaque noyau 24 est indépendant. Ainsi, en référence à la figure 5, les deux noyaux 24 illustrés sont à des hauteurs différentes. En l'espèce, un écart δ, plus ou moins grand et pouvant être nul, est ainsi visible sur la figure 5 entre les deux noyaux 24 représentés. Ainsi, cet écart δ est présent entre les extrémités libre 24A du noyau venant au contact de la surface interne biseautée 22A du godet 22. Cet écart est également présent au niveau des paliers 30. Cette différence de hauteur est liée à la différence de compression des deux ressorts 34. Cet écart peut être dû par exemple à des différences entre les godets 22, en particulier à cause de leurs tolérances de fabrication.

Le dispositif comporte en outre un flasque 39 et une plaque de réduction 40. Comme mieux visible sur les figures 5 à 7, le flasque 39 est destiné à rester sur la base 18 pour maintenir le godet 22, tandis que la plaque de réduction 40 est destinée à être retirée après réalisation du bâton de produit cosmétique 10 pour permettre la fixation de ce dernier à un mécanisme distributeur 16 avant de venir démouler le bâton de produit cosmétique 10 du godet 22.

Le flasque 39 est maintenu à la base 18 par exemple par aimantation.

La plaque de réduction 40 peut être disposée et centrée sur le flasque 39 par un doigt de centrage 42 fixé à la plaque de réduction 40. En l'espèce, il y a autant de doigt de centrage 42 qu'il y a de noyau 24 et de godets 22. En effet, comme mieux visible sur la figure 5, le doigt 42 vient s'insérer au travers du flasque 39 dans le godet 22. Le doigt 42 est bien entendu pourvu d'une ouverture débouchante 42' permettant le passage du noyau 24 au travers du doigt 42 et le remplissage avec la composition A destinée à former la gaine 14.

La plaque de réduction 40 peut être maintenue au flasque 39 par aimantation.

Afin de s'assurer du bon positionnement du noyau 24 dans le godet 22, le dispositif, et en particulier, le bloc 28 peut comporter en outre un moyen de guidage en translation et de centrage 36 du noyau 24 dans le godet 22.

Le moyen de guidage en translation et de centrage 36 comporte un ou plusieurs anneaux de guidage en translation et de centrage 44 destinés à s'insérer dans des logements 46 pourvus sur le flasque 39. En l'espèce, il peut être prévu deux anneaux de guidage en translation et de centrage 44 destinés à s'insérer dans deux logements 46 correspondants. Ces anneaux 44 permettent de centrer le bloc 28 contre la base 18 (par l'intermédiaire du flasque 39 et de la plaque de réduction 40). Les anneaux de guidage en translation et de centrage 44 s'insèrent et s'ajustent dans les logements 46 légèrement tronconique pour faciliter le guidage et le centrage. L'anneau 44 peut être en métal et est maintenu à la base 18 par l'intermédiaire du flasque 39 au moyen d'aimants 47 positionnés sur le flasque 39 en regard des logements 46 de la plaque de réduction 40, le bloc 28 étant alors maintenu fixement contre la base 18.

Le moyen de guidage en translation et de centrage 36 peut être escamotable, permettant ainsi de s'escamoter entre la position insérée (figure 3B) et la position retirée (figure 3A) du noyau 24.

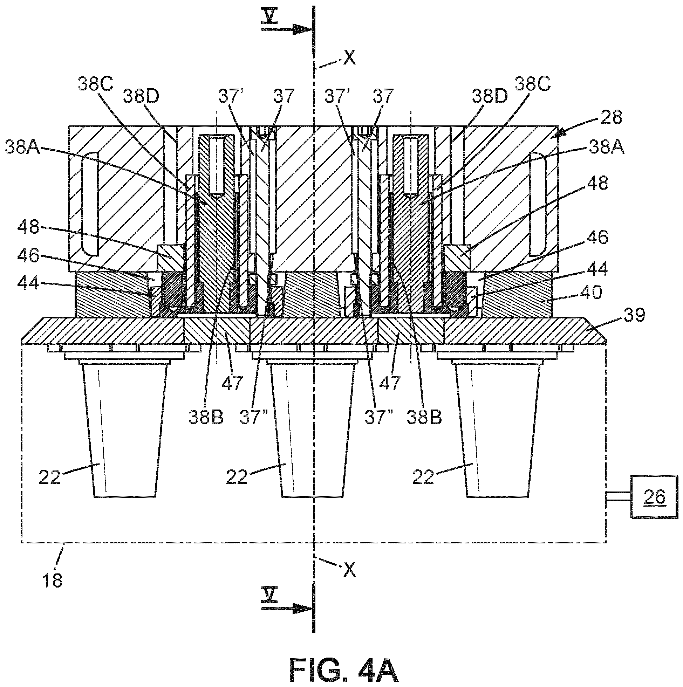

Par ailleurs, les anneaux 44 sont magnétiques de sorte que le moyen de guidage en translation et de centrage 36 peut être maintenu en position escamotée par rapport au bloc 28 par effet magnétique à l'aide d'aimants 48 pourvus sur le bloc 28 et/ou sur le moyen de guidage en translation et de centrage 36, comme mieux visible sur la figure 4A.

Lorsque le noyau 24 passe de la position retirée à la position insérée, le bloc 28 est approché de la base 18, en l'espèce de la plaque de réduction 40, de sorte que chaque noyau 24 s'insère progressivement dans le godet 22 correspondant.

Dès lors que les anneaux 44 viennent s'emmancher dans les logements 46, le moyen de guidage en translation et de centrage 36 étant préalablement escamoté, le noyau 24 a atteint sa position insérée dans le godet 22 (figure 4A).

A cet effet, le moyen de guidage en translation et de centrage 36 peut comporter un ensemble coulissant 38, plus précisément une paire d'ensembles coulissants 38. Chaque ensemble coulissant est formé d'une tige coulissante 38A montée coulissante en translation dans un palier de guidage 38C encastré dans le bloc 28, par l'intermédiaire de glissière 38B ou autres roulements ou cages à billes.

En référence aux figures 4A à 4C, le bloc 28 comporte un logement 38D dans lequel le palier de guidage 38C est encastré et par rapport auquel la tige 38A est apte à coulisser le long de l'axe longitudinal X-X; ainsi la tige 38A peut venir s'escamoter à l'intérieur du bloc 28 par coulissement.

Nous allons à présent décrire les étapes du procédé de fabrication d'un produit cosmétique 10 selon l'invention. Ne seront pas décrit les moyens d'amenée et de coulage ou d'injection des compositions A et B, ces moyens étant du type connu. Le procédé permet ainsi de fabriquer un bâton de produit cosmétique 10, notamment de rouge à lèvre, comprenant deux compositions A et B, s'étendant le long d'un axe longitudinal (X-X), mais également un bâton de produit cosmétique, notamment de rouge à lèvre, du type coeur-gaine. Pour toute la suite, la description est faite d'un procédé pour fabriquer un bâton de produit cosmétique 10 du type coeur-gaine, mais il doit être entendu qu'elle n'est pas limitée à ce type de bâton de produit cosmétique, mais peut être appliquée à tout type de bâton de produit cosmétique 10 comprenant deux compositions A et B.

Dans une étape E1, le moyen de guidage en translation et de centrage 36 est escamoté et les noyaux 24 sont préalablement lubrifiés avec un matériau lubrifiant permettant d'éviter que les compositions A et B n'y adhèrent. A cet effet, les noyaux 24 sont enduits de silicone. Afin de permettre le contact entre l'extrémité libre 24A du noyau 24 et la surface interne biseautée 22A du godet 22, il peut être prévu que l'extrémité libre 24A du noyau ne soit pas recouverte de silicone. L'enduction peut être réalisée par pulvérisation.

Dans une étape E2, la plaque de réduction 40 (et les doigts de centrage 42 qui y sont fixés) est mise en place sur le flasque 39 lui-même disposé sur la base 18.

Dans une étape E3, le bloc 28 est amené au-dessus de la base 18, en l'espèce au-dessus de la plaque de réduction 40, par exemple au moyen d'un bras robotisé (non illustré).

Dans une étape E4, le bloc 28 est alors descendu selon l'axe longitudinal X-X pour approcher le moyen de guidage en translation et de centrage 36 de la base 28, en l'espèce de la plaque de réduction 40, chaque noyau 24 venant s'insérer progressivement le godet 22 correspondant. Le rapprochement du bloc 28 s'arrête lorsque les anneaux de guidage en translation et de centrage 44 du moyen de guidage en translation et de centrage 36 sont en position dans les logements 46.

Les anneaux de guidage en translation et de centrage 44 s'insèrent de manière ajusté dans les logements 46 et sont maintenus par aimantation contre le flasque 39, le bloc 28 étant alors maintenu fixement contre la base 18. Chaque noyau 24 est alors au contact de la surface interne biseautée 22A du godet 22 sans déformer ce dernier, grâce au moyen d'ajustement de pression 25 correspondant. Chaque noyau 24 entre en contact avec la surface interne biseautée 22A du godet 22 avant la fin du rapprochement du bloc 28 (figures 3B, 4A et 5).

Une fois le noyau 24 mis en place à l'intérieur du godet 22, c'est-à-dire en position insérée, on amène dans une étape E5, une tête de remplissage (non illustrée) en coopération avec ouverture débouchante 42' prévue dans le godet 22 et on coule ou injecte à l'intérieur de la cavité C (volume disponible entre le noyau 24 et le godet 22) une quantité de produit de composition A correspondant sensiblement au volume de ladite cavité C (figure 5). Lors de cette étape de coulage ou d'injection, le produit de composition A pénètre à l'intérieur de la cavité C, chasse l'air initialement présent dans celle-ci et vient en contact de la surface en relief S' du godet 22.

On pourra éventuellement prévoir une étape de préchauffage ou de refroidissement (étape E6) du godet 22 réalisée avant l'étape de coulage ou d'injection de la composition A.

Il s'en suit une étape de refroidissement et de solidification (étape E7) au moins partielle de la composition A ainsi coulée. Après refroidissement et solidification au moins partielle, le produit cosmétique coulé à l'intérieur de la cavité C adhère au godet 22 au niveau de sa zone de remplissage par contact avec la paroi périphérique et le fond du godet 22. Il peut être prévu que l'ouverture débouchante 42' pourvue dans les doigts 42 soit légèrement conique se rétrécissant vers le bas du bâton de cosmétique 10, i.e. l'extrémité 10B du bâton de cosmétique 10. Cela permet de réduire le diamètre du bâton du bâton de cosmétique 10 afin de s'adapter sur le mécanisme de distribution 16 et de manière inhérente cela joue aussi un rôle sur le maintien de la gaine au moment du retrait du noyau.

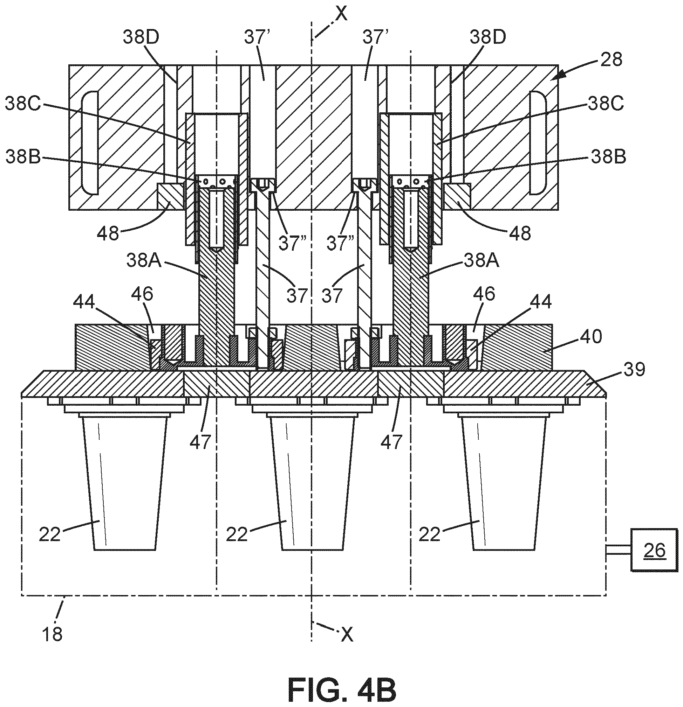

Le bloc 28 est alors remonté le long de l'axe longitudinal X-X (étape E8), les aimants 48 sont écartés des moyens de guidage en translation et de centrage 36 qui s'étirent progressivement, les noyaux 24 étant encore dans les godets 22, jusqu'à être entièrement déployés, les noyaux 24 étant alors hors des godets 22. La position entièrement déployée des moyens de guidage en translation et de centrage 36 est définie par une butée des ensembles coulissant 38. A cet effet, le dispositif peut comporter des vis 37 qui sont aptes à coulisser dans des alésages 37' prévus dans le bloc 28 et à venir en butée contre un épaulement 37" prévu dans les alésages 37' et qui sont vissées aux anneaux de guidage en translation et de centrage 44. Lorsque les vis 37 arrivent en butée dans les alésages 37' contre les épaulements 37" (voir figure 4B), les anneaux de guidage en translation et de centrage 44 sont encore logés dans les logements 46.

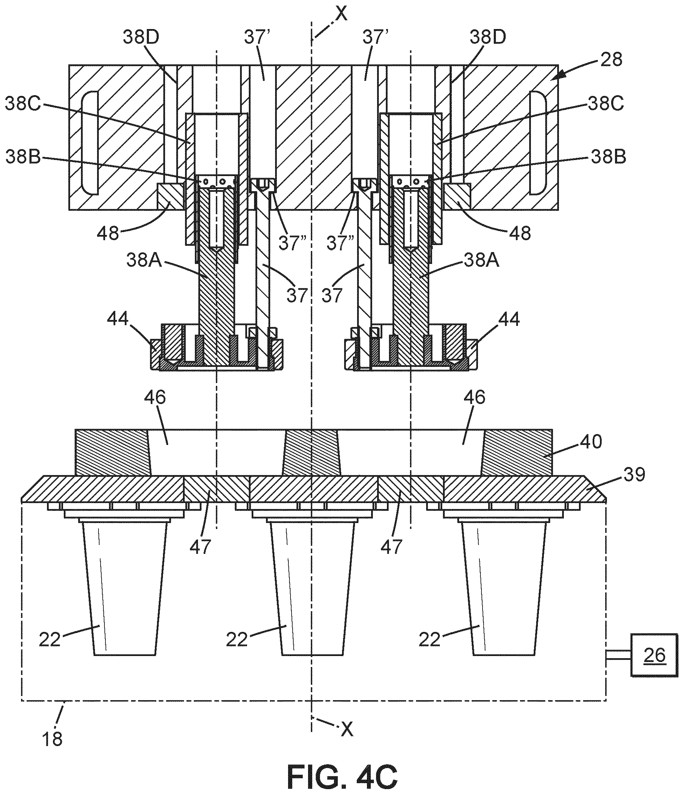

Tandis que le bloc 28 continue d'être écarté, jusqu'à ce que les noyaux 24 ont atteint leur position retirée, le long de l'axe longitudinal X-X de la base 18 (en l'espèce de la plaque de réduction 10), les vis 37 étant en butée, les anneaux de guidage en translation et de centrage 44 se retirent des logements 46 par l'intermédiaire des vis 37 (voir figure 4C). Il peut être prévu que les aimants 48 maintenant la position escamotée ont une force magnétique plus faible que les aimants 47 maintenant l'anneau 44 dans le logement 46. Le moyen de guidage en translation et de centrage 36 permet une extraction rectiligne des noyaux 24 permettant d'éviter d'endommager la gaine 14 jusqu'à la position retirée (figures 3A, 4C et 6A).

Lorsque le noyau 24 est retiré du godet, la surface interne biseautée 22A du godet 22 qui est au contact de l'extrémité libre 24A du noyau 24 peut par effet ventouse être entraînée avec le noyau 24. L'intérieur de la gaine 14 duquel le noyau 24 est retiré, pourrait alors être endommagé. Pour éviter cet inconvénient, une mise à l'air du noyau 24 est réalisée grâce au canal d'évacuation d'air 24' (ou évent) débouchant à l'extrémité libre 24A du noyau 24 .

Dès lors que les noyaux 24 sont en position retirée, le bloc 28 est écarté de la base 28 par des moyens de déplacement (non illustrés). L'enduction de silicone précitée est alors réitérée sur les noyaux 24 (étape E1). Le moyen de guidage en translation et de centrage 36 est alors rétracté en position escamotée par des moyens de rétractation (non illustrés).

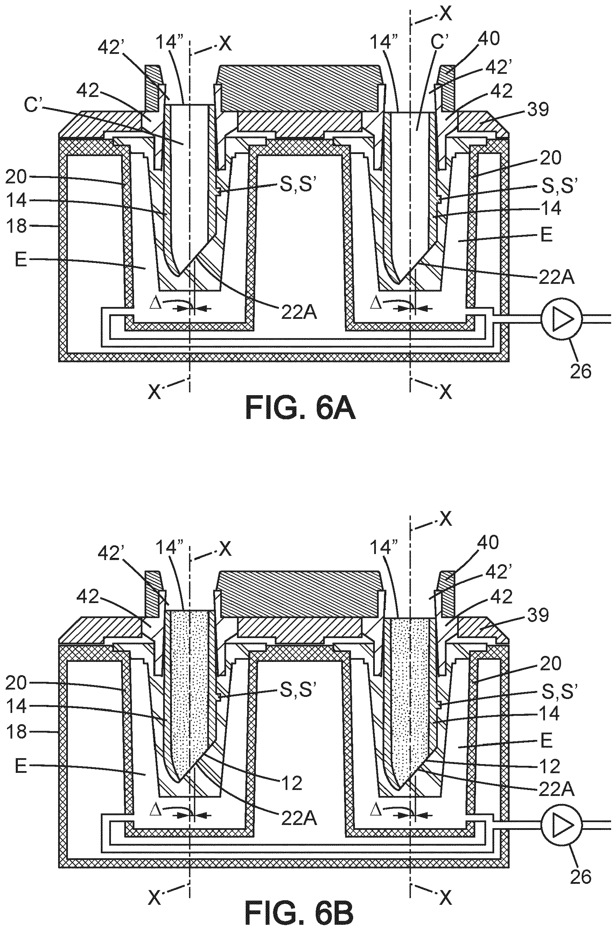

Lorsque le noyau 24 est retiré du godet 22 (voir figure 6A), il laisse la place à une cavité C' formée à l'intérieur de la gaine 14. Cette cavité C' présente une ouverture débouchante 14" qui peut alors être remplie avec la composition B.

On amène alors (étape E9) une seconde tête de remplissage (non illustrée) en coopération avec ouverture débouchante 14" et on coule ou injecte à l'intérieur de la seconde cavité C', une quantité d'un second produit composition B correspondant sensiblement au volume de ladite seconde cavité C'. Lors de cette étape de coulage ou d'injection, le second produit composition B pénètre à l'intérieur de la seconde cavité C' et vient adhérer à la gaine 14 (composition A) au niveau de leur interface de contact (voir figure 6B).

De préférence, le second produit cosmétique (composition B) coulé à l'intérieur de la gaine 14 présente une température de fusion inférieure ou égale à celle du premier produit cosmétique (composition A). De cette manière, on évite que le second produit cosmétique (composition B) coulé à l'intérieur de la gaine 14 ne fasse fondre le premier produit cosmétique (composition A). Dans ce cas, l'adhérence entre le coeur 12 et la gaine 14 peut être obtenue par frottement ou réaction chimique, entre les compositions A et B, localisée à l'interface du coeur 12 et de la gaine 14.

A défaut, il peut être prévu que le second produit cosmétique (composition B) coulé à l'intérieur de la gaine 14 présente une température de fusion légèrement supérieure à celle du premier produit cosmétique (composition A). De cette manière, on lorsque le second produit cosmétique (composition B) est coulé à l'intérieur de la gaine 14 il fait fondre localement le premier produit cosmétique (composition A). Dans ce cas, l'adhérence entre le coeur 12 et la gaine 14 peut être obtenue par fusion/mélange, entre les compositions A et B, localisée à l'interface du coeur 12 et de la gaine 14.

Il s'en suit une nouvelle étape de refroidissement et de solidification (étape E10) du second produit cosmétique (composition B) coulé. Le second produit cosmétique (composition B) coulé à l'intérieur de la seconde cavité C' adhère alors au premier produit cosmétique (composition A) coulé au cours du premier cycle, de manière à former avec ledit produit cosmétique (composition A) coulé au cours du premier cycle un ensemble solidaire, à savoir le bâton de produit cosmétique 10 selon l'invention.

Avant de démouler le bâton de produit cosmétique 10 du godet 22, la plaque de réduction 40, et donc le doigt de centrage 42 fixé à la plaque de réduction 40, est retirée dans une étape E11, pour permettre la fixation du bâton de produit cosmétique 10 à un mécanisme distributeur 16, tel qu'illustré sur la figure 7. La manipulation de la plaque de réduction peut être faite par un bras robotisé (non représenté sur les figures).

Afin de pouvoir extraire le bâton de produit cosmétique 10 du godet 22 sans abimer la portion de surface décorative S en relief, le moyen 26 pour déformer le godet 22 est actionné pour déformer chacun des six godets 22 (étape E12), tel qu'illustré sur la figure 7. Sous l'effet de l'aspiration d'air, l'espace E présent entre le godet 22 et la cavité 20 est réduit en un espace E' et le godet 22 se déforme grâce à son élasticité sous l'effet de cette pression. La surface en relief S' du godet 22 est ainsi également retirée de la surface en relief S formée sur la gaine 4 du bâton de produit cosmétique 10.

Dès lors que le godet 22 est déformé, il s'écarte de la gaine 14 et du coeur 12 et le démoulage du bâton de produit cosmétique 10 peut être réalisé sans détérioration du bâton de produit cosmétique 10, et en particulier sans détérioration de la surface en relief S.

Après démoulage, la surface en relief S forme un motif décoratif en relief sur la face bâton de produit cosmétique 10 obtenu qui est exposé à l'utilisateur / l'utilisatrice.

Le cycle de fabrication peut alors reprendre au début du procédé décrit ci-dessus (étapes E1 à E12).

Une machine à plateaux rotatifs peut être utilisée pour mettre en oeuvre ce procédé, les bases 18 se déplaçant alors de poste en poste pour la réalisation de chaque étape E1 à E12.

## Revendications

1. Bâton de produit cosmétique, notamment de rouge à lèvre, comprenant deux compositions A et B s'étendant le long d'un axe longitudinal (X-X), les compositions A et B formant respectivement deux éléments (14, 12) constitutifs du bâton de produit cosmétique, le bâton de produit cosmétique (10) ayant une surface latérale (10C) présentant une portion de surface décorative en relief (S) formant au moins une contre-dépouille par rapport à un démoulage selon l'axe longitudinal (X-X) du bâton de produit cosmétique (10).

2. Bâton de produit cosmétique selon la revendication 1, dans lequel le bâton de produit cosmétique est du type coeur-gaine, les deux compositions A et B étant disposées l'une par rapport à l'autre pour former un coeur (12) entouré d'une gaine (14) le long de l'axe longitudinal (X-X), la gaine (14) ayant ladite surface latérale (10C) présentant la portion de surface décorative en relief (S).

3. Bâton de produit cosmétique selon la revendication 1 ou la revendication 2, ayant une extrémité libre biseautée (10A).

4. Bâton de produit cosmétique selon la revendication 2 en combinaison avec la revendication 3, dans lequel la gaine (14) présente au niveau de l'extrémité libre biseautée (10A) une épaisseur (14') constante autour du coeur (12).

5. Bâton de produit cosmétique selon la revendication 3, dans lequel au niveau de l'extrémité libre biseautée (10A) le coeur (12) est concentrique avec la gaine (14).

6. Bâton de produit cosmétique selon l'une quelconque des revendications 1 à 4, dans lequel la portion de surface décorative en relief (S) est en saillie ou en creux.

7. Procédé de fabrication d'un bâton de produit cosmétique, notamment de rouge à lèvre, comprenant deux compositions A et B, s'étendant le long d'un axe longitudinal (X-X), le bâton ayant une surface latérale (10C) présentant une portion de surface décorative en relief (S), où le procédé met en oeuvre :
- une étape (E4) consistant à mettre en place un noyau (24) dans un godet (22) présentant une portion de surface en relief (S'),
- une étape (E5) consistant à couler ou injecter une composition A entre le noyau (24) et le godet (22) de sorte à former un premier élément (14),
- une étape (E8) consistant à retirer le noyau (24) du godet (22),
- une étape (E9) consistant à couler ou injecter une composition B à l'emplacement laissé libre par le noyau (24) de sorte de former un deuxième élément (12), formant ainsi un bâton de produit cosmétique (10), et
- une étape (E12) consistant à déformer le godet (22) de sorte de démouler le bâton de produit cosmétique (10) .

8. Procédé permettant de fabriquer un bâton de produit cosmétique, notamment de rouge à lèvre, du type coeur-gaine, comprenant deux compositions A et B, s'étendant le long d'un axe longitudinal (X-X), les deux compositions A et B étant disposées l'une par rapport à l'autre pour former un coeur (12) entouré d'une gaine (14) le long de l'axe longitudinal (X-X), la gaine (14) ayant une surface latérale (10C) présentant la portion de surface décorative en relief (S), ou le procédé met en oeuvre :
- une étape (E4) consistant à mettre en place un noyau (24) dans un godet (22) présentant une portion de surface en relief (S'),
- une étape (E5) consistant à couler ou injecter une composition A entre le noyau (24) et le godet (22) de sorte à former la gaine (14),
- une étape (E8) consistant à retirer le noyau (24) du godet (22),
- une étape (E9) consistant à couler ou injecter une composition B dans la gaine (14) de sorte de former le coeur (12) entouré par la gaine (14), formant ainsi le bâton de produit cosmétique (10) du type coeur-gaine, et
- une étape (E12) consistant à déformer le godet (22) de sorte de démouler le bâton de produit cosmétique (10) .

9. Procédé de fabrication d'un bâton de produit cosmétique selon la revendication 7 ou la revendication 8, comprenant en outre une étape (E1) consistant à lubrifier le noyau (24).

10. Dispositif de fabrication d'un bâton de produit cosmétique, notamment de rouge à lèvre, s'étendant le long d'un axe longitudinal (X-X), le bâton ayant une surface latérale (10C) présentant une portion de surface décorative en relief (S), le dispositif comportant :
- une base (18) comportant une cavité (20) formée dans la base (18),
- un godet (22) élastique reçu dans la cavité (20), le godet (22) présentant une surface en relief (S'),
- un moyen pour déformer le godet (22) afin de pouvoir extraire un bâton de produit cosmétique (10) du godet (22),
- et un noyau (24) mobile en translation entre une position insérée dans laquelle le noyau (24) est positionné dans le godet (22), le noyau (24) venant en contact avec le fond du godet (22) de façon à permettre de couler ou injecter une composition A entre le noyau (24) et le godet (22), et une position retirée dans laquelle le noyau (24) est retiré du godet (22) de façon à permettre de couler ou injecter une composition B à l'emplacement laissé libre par le noyau (24).

11. Dispositif selon la revendication 10, dans lequel le bâton de produit cosmétique, notamment de rouge à lèvre, est du type coeur-gaine, les deux compositions A et B étant disposées l'une par rapport à l'autre pour former un coeur (12) entouré d'une gaine (14) le long de l'axe longitudinal (X-X), la gaine (14) ayant une surface latérale (10C) présentant la portion de surface décorative en relief (S).

12. Dispositif selon la revendication 10 ou la revendication 11, dans lequel
- le godet (22) est d'une taille inférieure à celle de la cavité (20) de façon à définir un espace (E) entre le godet (22) et la cavité (20), et
- ledit godet (22) comporte un moyen (26) pour mettre en dépression l'espace (E).

13. Dispositif selon l'une quelconque des revendications 10 à 12, dans lequel le noyau (24) présente un canal d'évacuation d'air (24') débouchant d'une part sur l'extrémité du noyau (24) en contact avec la surface interne du godet (22) et d'autre part dans une portion du noyau (24) hors du godet (22) lorsque le noyau (24) est inséré dans ledit godet (22).

14. Dispositif selon l'une quelconque des revendications 10 à 13, comprenant en outre un moyen d'ajustement de pression (25) du noyau (24) dans le godet (22) .

15. Dispositif selon la revendication précédente, dans lequel le moyen d'ajustement de pression (25) comporte un ressort (34).

16. Dispositif selon l'une quelconque des revendications 10 à 14, comprenant en outre un moyen de guidage en translation et de centrage (36) du noyau (24) dans le godet (22).

17. Dispositif selon la revendication précédente, dans lequel le moyen de guidage en translation et de centrage (36) est escamotable.

18. Dispositif selon la revendication 16 ou la revendication 17, dans lequel le noyau (24) et le moyen de guidage en translation et de centrage (36) sont montés sur un bloc (28).

19. Dispositif selon la revendication 17 ou la revendication 18, comprenant en outre un moyen d'aimantation (48) permettant de maintenir le moyen de guidage en translation et de centrage (36) en position escamotée.

20. Dispositif selon l'une quelconque des revendications 16 à 19, dans lequel le moyen de guidage en translation et de centrage (36) comporte une bague de centrage (44) apte à coopérer avec un logement (46) pourvu dans la base (18).

21. Dispositif selon l'une quelconque des revendications 10 à 20, comprenant une pluralité de cavités (20), une pluralité de godets (22) élastiques présentant chacun une surface en relief (S') et une pluralité de noyaux (24), dans lequel la pluralité de cavités (20) est formée dans la base (18), dans lequel chacun des godets (22) est reçu dans chaque cavité (20), et dans lequel chacun des noyaux (24) est mobile en translation entre une position insérée dans laquelle les noyaux (24) sont positionnés dans les godets (22) et une position retirée dans laquelle les noyaux (24) sont retirés des godets (22).

## Patentansprüche

1. Kosmetikproduktstift, insbesondere Lippenstift, aufweisend zwei Zusammensetzungen A und B, die sich entlang einer Längsachse (X-X) erstrecken, wobei die zwei Zusammensetzungen A und B jeweils zwei Bestandteile (14, 12) des Kosmetikproduktstifts bilden, wobei der Kosmetikproduktstift (10) eine Seitenfläche (10C) hat, die einen reliefartigen dekorativen Oberflächenabschnitt (S) aufweist, der mindestens eine Gegenformschräge bildet in Hinblick auf Entnahme aus der Form in Richtung der Längsachse (X-X) des Kosmetikproduktstifts (10).

2. Kosmetikproduktstift nach Anspruch 1, in welchem der Kosmetikproduktstift vom Typ Herz-Mantel ist, wobei die zwei Zusammensetzungen A und B zueinander angeordnet sind, um entlang der Längsachse (X-X) ein von einem Mantel (14) umgebenes Herz (12) zu bilden, wobei der Mantel (14) die Seitenfläche (10C) hat, die den reliefartigen dekorativen Oberflächenabschnitt (S) aufweist.

3. Kosmetikproduktstift nach Anspruch 1 oder Anspruch 2, aufweisend ein abgeschrägtes freies Ende (10A).

4. Kosmetikproduktstift nach Anspruch 2 in Kombination mit Anspruch 3, in welchem der Mantel (14) an dem abgeschrägten freien Ende (10A) eine konstante Dicke (14') um das Herz (12) herum aufweist.

5. Kosmetikproduktstift nach Anspruch 3, in welchem an dem abgeschrägten freien Ende (10A) das Herz (12) konzentrisch mit dem Mantel (14) ist.

6. Kosmetikproduktstift nach einem der Ansprüche 1 bis 4, in welchem der reliefartige dekorative Oberflächenabschnitt (S) vorspringend oder eingetieft ist.

7. Verfahren zur Herstellung eines Kosmetikproduktstifts, insbesondere eines Lippenstifts, der zwei Zusammensetzungen A und B aufweist, die sich entlang einer Längsachse (X-X) erstrecken, wobei der Stift eine Seitenfläche (10C) hat, die einen reliefartigen dekorativen Oberflächenabschnitt (S) aufweist, wobei das Verfahren ausführt:
- einen Schritt (E4), der darin besteht, einen Kern (24) in einem mit einem reliefartigen Oberflächenabschnitt (S') versehenen Topf (22) anzuordnen,
- einen Schritt (ES), der darin besteht, eine Zusammensetzung A zwischen den Kern (24) und den Topf (22) zu gießen oder spritzen, um so einen ersten Bestandteil (14) zu bilden,
- einen Schritt (E8), der darin besteht, den Kern (24) aus dem Topf (22) herauszuziehen,
- einen Schritt (E9), der darin besteht, eine Zusammensetzung B an den von dem Kern (24) frei gelassenen Ort zu gießen oder spritzen, um so einen zweiten Bestandteil (12) zu bilden und damit einen Kosmetikproduktstift (10) zu bilden, und
- einen Schritt (E12), der darin besteht, den Topf (22) zu verformen, um den Kosmetikproduktstift (10) zu entformen.

8. Verfahren, das erlaubt, einen Kosmetikproduktstift, insbesondere Lippenstift, des Typs Herz-Mantel herzustellen, der zwei Zusammensetzungen A und B aufweist, die sich entlang einer Längsachse (X-X) erstecken, wobei die zwei Zusammensetzungen zueinander angeordnet sind, um entlang der Längsachse (X-X) ein von einem Mantel (14) umgebenes Herz (12) zu bilden, wobei der Mantel (14) eine Seitenfläche (10C) hat, die einen reliefartigen dekorativen Oberflächenabschnitt (S) aufweist, wobei das Verfahren ausführt:
- einen Schritt (E4), der darin besteht, einen Kern (24) in einem mit einem reliefartigen Oberflächenabschnitt (S') versehenen Topf (22) anzuordnen,
- einen Schritt (E5), der darin besteht, eine Zusammensetzung A zwischen den Kern (24) und den Topf (22) zu gießen oder spritzen, um so den Mantel (14) zu bilden,
- einen Schritt (E8), der darin besteht, den Kern (24) aus dem Topf (22) herauszuziehen,
- einen Schritt (E9), der darin besteht, eine Zusammensetzung B in den Mantel (14) zu gießen oder spritzen, um so das von dem Mantel (14) umgebene Herz (12) zu bilden und damit den Kosmetikproduktstift (10) des Typs Herz-Mantel zu bilden, und
- einen Schritt (E12), der darin besteht, den Topf (22) zu verformen, um den Kosmetikproduktstift (10) zu entformen.

9. Verfahren zur Herstellung eines Kosmetikproduktstifts nach Anspruch 7 oder Anspruch 8, aufweisend ferner einen Schritt (E1), der darin besteht, den Kern (24) zu schmieren.

10. Vorrichtung zur Herstellung eines Kosmetikproduktstifts, insbesondere eines Lippenstifts, der sich entlang einer Längsachse (X-X) erstreckt, wobei der Stift eine Seitenfläche (10C) hat, die einen reliefartigen dekorativen Oberflächenabschnitt (S) aufweist, wobei die Vorrichtung aufweist:
- eine Basis (18) mit einem in der Basis (18) gebildeten Hohlraum (20),
- einen Topf (22), der elastisch in dem Hohlraum (20) aufgenommen ist, wobei der Topf (22) eine reliefartige Oberfläche (S') aufweist,
- eine Einrichtung zum Verformen des Topfs (22), um einen Kosmetikproduktstift (10) aus dem Topf (22) nehmen zu können,
und einen Kern (24), der verschiebbar ist zwischen einer eingesetzten Position, in der der Kern (24) in dem Topf (22) angeordnet ist und der Kern (24) mit dem Boden des Topfs (22) in Kontakt ist, um so zu erlauben, eine Zusammensetzung A zwischen den Kern (24) und den Topf (22) zu gießen oder spritzen, und einer herausgezogenen Position, in der der Kern (24) aus dem Topf (22) herausgezogen ist, um so zu erlauben, eine Zusammensetzung B an den von dem Kern (24) frei gelassenen Ort zu gießen oder spritzen.

11. Vorrichtung nach Anspruch 10, in welcher der Kosmetikproduktstift, insbesondere Lippenstift, des Typs Herz-Mantel ist, wobei die zwei Zusammensetzungen zueinander angeordnet sind, um entlang der Längsachse (X-X) ein von einem Mantel (14) umgebenes Herz (12) zu bilden, wobei der Mantel (14) eine Seitenfläche (10C) hat, die einen reliefartigen dekorativen Oberflächenabschnitt (S) aufweist.

12. Vorrichtung nach Anspruch 10 oder Anspruch 11, in welcher
- der Topf (22) eine Größe hat, die geringer als die des Hohlraums (20) ist, um so einen Spalt (E) zwischen dem Topf (22) und dem Hohlraum (20) zu definieren, und
- der Topf (22) eine Einrichtung aufweist, um den Spalt (E) unter Unterdruck zu setzen.

13. Vorrichtung nach einem der Ansprüche 10 bis 12, in welcher der Kern (24) einen Luftauslasskanal (24') aufweist, der einerseits an dem Ende des Kerns (24), das mit der Innenfläche des Topfs (24) in Kontakt ist, ausmündet und andererseits in einen Teil des Kerns (24) mündet, der, wenn der Kern (24) in den Topf (22) eingesetzt ist, außerhalb des Topfs (22) ist.

14. Vorrichtung nach einem der Ansprüche 10 bis 13, aufweisend ferner eine Druckanpassungseinrichtung (25) zum Anpassen eines Hineindrückens des Kerns (24) in den Topf (22).

15. Vorrichtung nach dem vorstehenden Anspruch, in welcher die Druckanpassungseinrichtung (25) eine Feder (34) aufweist.

16. Vorrichtung nach einem der Ansprüche 10 bis 14, aufweisend ferner eine Verschiebungsführungs- und Zentriereinrichtung (36) zur Führung der Verschiebung und zur Zentrierung des Kerns (24) in den Topf (22).

17. Vorrichtung nach dem vorstehenden Anspruch, in welcher die Verschiebungsführungs- und Zentriereinrichtung (36) versenkbar ist.

18. Vorrichtung nach Anspruch 16 oder Anspruch 17, in welcher der Kern (24) und die Verschiebungsführungs- und Zentriereinrichtung (36) an einem Block (28) angebracht sind.

19. Vorrichtung nach Anspruch 17 oder Anspruch 18, aufweisend ferner eine Magnetisierungseinrichtung (48), die erlaubt, die Verschiebungsführungs- und Zentriereinrichtung (36) in einer versenkten Position zu halten.

20. Vorrichtung nach einem der Ansprüche 16 bis 19, in welcher die Verschiebungsführungs- und Zentriereinrichtung (36) einen Zentrierungsring (44) aufweist, der fähig ist, mit einer in der Basis (18) vorgesehenen Aufnahme (46) zusammenzuwirken.

21. Vorrichtung nach einem der Ansprüche 10 bis 20, aufweisend mehrere Hohlräume (20), mehrere elastische Töpfe (22) mit jeweils einer reliefartigen Oberfläche (S') und mehrere Kerne (24), in welcher die mehreren Hohlräume (20) in der Basis (18) gebildet sind, in welcher jeweils einer der Töpfe (22) in einem der Hohlräume (20) aufgenommen ist, und in welcher jeder der Kerne (24) verschiebbar ist zwischen einer eingesetzten Position, in der die Kerne (24) in den Töpfen (22) angeordnet sind, und einer herausgezogenen Position, in der die Kerne (24) aus den Töpfen (22) herausgezogen sind.

## Claims

1. Stick of cosmetic product, in particular of lipstick, comprising two compositions A and B extending along a longitudinal axis (X-X), the compositions A and B forming respectively two constituent elements (14, 12) of the stick of cosmetic product, the stick of cosmetic product (10) having a side surface (10C) presenting a decorative surface portion in relief (S) forming at least one undercut when demolding along the longitudinal axis (X-X) of the stick of cosmetic product (10).

2. Stick of cosmetic product according to claim 1, wherein the stick of cosmetic product is of the central part-sheath type, the two compositions A and B being arranged relative to one another to form a central part (12) surrounded by a sheath (14) along the longitudinal axis (X-X), the sheath (14) having said side surface (10C) presenting the decorative surface portion in relief (S).

3. Stick of cosmetic product according to claim 1 or 2, having a beveled free end (10A).

4. Stick of cosmetic product according to claim 2 in combination with claim 3, wherein the sheath (14) has, at the beveled free end (10A), a constant thickness (14') around the central part (12).

5. Stick of cosmetic product according to claim 3, wherein at the beveled free end (10A), the central part (12) is concentric with the sheath (14).

6. Stick of cosmetic product according to any one of claims 1 to 4, wherein the decorative surface portion in relief (S) is protruding or recessed.

7. Method for manufacturing a stick of cosmetic product, in particular of lipstick, comprising two compositions A and B, extending along a longitudinal axis (X-X), the stick having a side surface (10C) presenting a decorative surface portion in relief (S), wherein the method implements:
- a step (E4) of placing a core (24) in a cup (22) having a surface portion in relief (S'),
- a step (E5) of pouring or injecting a composition A between the core (24) and the cup (22) so as to form a first member (14),
- a step (E8) of removing the core (24) from the cup (22),
- a step (E9) of pouring or injecting a composition B at the place left free by the core (24) so as to form a second member (12), thereby forming a stick of cosmetic product (10), and
- a step (E12) of deforming the cup (22) so as to demold the stick of cosmetic product (10).

8. Method enabling the manufacture of a stick of cosmetic product, in particular of lipstick, of the central part-sheath type, the two compositions A and B being arranged relative to one another to form a central part (12) surrounded by a sheath (14) along the longitudinal axis (X-X), the sheath (14) having a side surface (10C) presenting the decorative surface portion in relief (S), wherein the method implements:
- a step (E4) of placing a core (24) in a cup (22) having a surface portion in relief (S'),
- a step (E5) of pouring or injecting a composition A between the core (24) and the cup (22) so as to form a sheath (14),
- a step (E8) of removing the core (24) from the cup (22),
- a step (E9) of pouring or injecting a composition B into the sheath (14) so as to form a central part (12) surrounded by the sheath (14), thereby forming a stick of cosmetic product (10) of the central part-sheath type, and
- a step (E12) of deforming the cup (22) so as to demold the stick of cosmetic product (10).

9. Method for manufacturing a stick of cosmetic product according to claim 7 or claim 8, further comprising a step (E1) of lubricating the core (24).

10. Device for manufacturing a stick of cosmetic product, in particular of lipstick, extending along a longitudinal axis (X-X), the stick having a side surface (10C) presenting a decorative surface portion in relief (S), the device comprising:
- a base (18) having a cavity (20) formed in the base (18),
- an elastic cup (22) received in the cavity (20), the cup (22) having a surface in relief (S'),
- a means for deforming the cup (22) in order to be able to extract a stick of cosmetic product (10) from the cup (22),
- and a core (24) movable in translation between an inserted position in which the core (24) is positioned in the cup (22), the core (24) coming into contact with the bottom of the cup (22) so as to enable pouring or injecting a composition A between the core (24) and the cup (22), and a withdrawn position in which the core (24) is withdrawn from the cup (22) so as to enable pouring or injecting a composition Bat the place left free by the core (24).

11. Device according to claim 10, wherein the stick of cosmetic product, in particular of lipstick, is of the central part-sheath type, the two compositions A and B being arranged relative to one another to form a central part (12) surrounded by a sheath (14) along the longitudinal axis (X-X), the sheath (14) having a side surface (10C) presenting the decorative surface portion in relief (S).

12. Device according to claim 10 or claim 11, wherein
- the cup (22) is of a size smaller than that of the cavity (20) so as to define a space (E) between the cup (22) and the cavity (20), and
- said cup (22) comprises means (26) for creating negative pressure in the space (E).

13. Device according to any one of claims 10 to 12, wherein the core (24) has an air outlet channel (24') leading between: the end of the core (24) in contact with the inner surface of the cup (22), and a portion of the core (24) outside the cup (22) when the core (24) is inserted into said cup (22).

14. Device according to any of claims 10 to 13, further comprising pressure adjustment means (25) for adjusting the pressure of the core (24) in the cup (22).

15. Device according to the preceding claim, wherein the pressure adjustment means (25) comprises a spring (34).

16. Device according to any one of claims 10 to 14, further comprising a means for guiding in translation and for centering (36) the core (24) in the cup (22).

17. Device according to the preceding claim, wherein the means for guiding in translation and for centering (36) is retractable.

18. Device according to claim 16 or claim 17, wherein the core (24) and the means for guiding in translation and for centering (36) are mounted on a block (28).

19. Device according to claim 17 or claim 18, further comprising a magnetization means (48) for maintaining in the retracted position the means for guiding in translation and for centering (36).

20. Device according to any one of claims 16 to 19, wherein the means for guiding in translation and for centering (36) comprises a centering ring (44) adapted to engage with a housing (46) provided in the base (18).

21. Device according to any one of claims 10 to 20, comprising a plurality of cavities (20), a plurality of elastic cups (22) each having a surface in relief (S'), and a plurality of cores (24), wherein the plurality of cavities (20) are formed in the base (18), wherein each of the cups (22) is received in each cavity (20), and wherein each of the cores (24) is movable in translation between an inserted position in which the cores (24) are positioned in the cups (22) and a withdrawn position in which the cores (24) are withdrawn from the cups (22).
